# EUROPEAN PATENT APPLICATION

(11) **EP 2 199 408 A1**
(43) Date of publication of application: **23.06.2010**
(21) Application number: 10001472.9
(22) Date of filing: 15.02.2006
(51) Int. Cl.: C12Q 1/68

(54) **Method of diagnosing type II diabetes mellitus using multilocus marker, polynucleotide including marker associated with type II diabetes mellitus, and microarray and diagnostic kit including the polynucleotide**

(30) Priority: 15.02.2005 KR 20050012418; 20.04.2005 KR 20050032739
(62) Divisional of application: 06715973.1
(71) Applicant: Samsung Electronics Co., Ltd., Suwon-si Gyeonggi-do 443-742 (KR)
(72) Inventor: Kim, Jae-Heup, Taean-eub Hwaseong-si Gyeonggi-do 445-774 (KR); Choi, Seung-Hak, Bundang-gu Seongnam-si Gyeonggi-do 463-738 (KR); Nam, Yun-Sun, Sujeong-gu Seongnam-si Gyeonggi-do 461-760 (KR); Lee, Yeon-Su, Goyang-si, Gyeonggi-do 411-704 (KR); Hwang, Jung-Joo, Suwon-si, Gyeonggi-do 443-769 (KR); Lee, Kyu-Sang, Suwon-si, Gyeonggi-do 443-706 (KR)
(74) Representative: Grünecker, Kinkeldey, Stockmair & Schwanhäusser Anwaltssozietät

(57) **Abstract**

Provided are a polynucleotide including a marker associated with type II diabetes mellitus and a method of diagnosing type II diabetes mellitus in an individual, which includes determining a nucleotide of a polymorphic site of at least one polynucleotide of Table 1 in the specification.

## Description

### Technical Field

The present invention relates to a method of diagnosing type II diabetes mellitus using a multilocus marker, a polynucleotide including a marker associated with type II diabetes mellitus, and a microarray and a diagnostic kit including the polynucleotide.

### Background Art

The genomes of all organisms undergo spontaneous mutation in the course of their continuing evolution, generating variant forms of progenitor nucleic acid sequences (Gusella, Ann. Rev. Biochem. 55, 831-854 (1986)). The variant forms may confer an evolutionary advantage or disadvantage, relative to a progenitor form, or may be neutral. In some instances, a variant form confers a lethal disadvantage and is not transmitted to subsequent generations of the organism. In other instances, a variant form confers an evolutionary advantage to the species and is eventually incorporated into the DNA of most members of the species and effectively becomes the progenitor form. In many instances, both progenitor and variant form(s) survive and co-exist in a species population. The coexistence of multiple forms of a sequence gives rise to polymorphisms.

Several different types of polymorphisms are known, including restriction fragment length polymorphisms (RFLPs), short tandem repeats (STRs), and single-nucleotide polymorphisms (SNPs). Among them, SNPs take the form of single-nucleotide variations between individuals of the same species. When SNPs occur in protein coding sequences, any one of the polymorphic forms may give rise to the expression of a defective or a variant protein. On the other hand, when SNPs occur in non-coding sequences, some of these polymorphisms may result in the expression of defective or variant proteins (e.g., as a result of defective splicing). Other SNPs have no phenotypic effects.

It is known that human SNPs occur at a frequency of 1 in about 300-1,000 bp. When such SNPs induce a phenotypic expression such as a disease, polynucleotides containing the SNPs can be used as primers or probes for diagnosis of a disease. Currently, research into the nucleotide sequences and functions of SNPs is being conducted by many research institutes. The nucleotide sequences and other experimental results of the identified human SNPs have been made into a database to be easily accessible.

Even though findings available to date show that specific SNPs exist on human genomes or cDNAs, phenotypic effects of such SNPs have not been revealed. Functions of most SNPs have not been disclosed yet.

It is known that 90-95% of total diabetes patients suffer type II diabetes mellitus. Type II diabetes mellitus is a disorder which is developed in persons who abnormally produce insulin or have low sensitivity to insulin, thereby resulting in large change in blood glucose level. When disorder of insulin secretion leads to the condition of type II diabetes mellitus, blood glucose cannot be transferred to body cells, which renders the conversion of food into energy difficult. It is known that genetic causes play a role in type II diabetes mellitus. Other risk factors of type II diabetes mellitus include age over 45, familial history of diabetes mellitus, obesity, hypertension, and high cholesterol level. Currently, diabetes mellitus is mainly diagnosed by measuring a pathological phenotypic change, i.e., blood glucose level, using fasting blood glucose (FSB) test, oral glucose tolerance test (OGTT), and the like [National Institute of Diabetes and Digestive and Kidney Diseases of the National Institutes of Health, http://www.niddk.nih.gov, 2003]. When diagnosis of type II diabetes mellitus is made, type II diabetes mellitus can be prevented or its onset can be delayed by exercise, special diet, body weight control, drug therapy, and the like. In this regard, it can be said that type II diabetes mellitus is a disease in which early diagnosis is highly desirable. Millenium Pharmaceuticals Inc. reported that diagnosis and prognosis of type II diabetes mellitus could be made based on genotypic variations present on HNF1 gene [PR newswire, Sept 1,1998]. Sequenom Inc. reported that FOXA2 (HNF3 β) gene was highly associated with type II diabetes mellitus [PR Newswire, Oct 28, 2003]. Even though there are reports about some genes associated with type II diabetes mellitus, research into the incidence of type II diabetes mellitus has been focused on specific genes of some chromosomes in specific populations. For this reason, research results may vary according to human species. Furthermore, all causative genes responsible for type II diabetes mellitus have not yet been identified. Diagnosis of type II diabetes mellitus by such a molecular biological technique is now uncommon. In addition, early diagnosis before incidence of type II diabetes mellitus is currently not feasible. Therefore, there is an increasing need to find new SNPs highly associated with type II diabetes mellitus and their related genes that are found in whole human genomes and to make an early diagnosis of type II diabetes mellitus using the SNPs and the related genes.

### Disclosure of Invention

### Technical Problem

The present invention provides a method of diagnosing type II diabetes mellitus using a multilocus marker.

The present invention also provides a polynucleotide including a marker associated with type II diabetes mellitus.

The present invention also provides a microarray including a substrate immobilized with the polynucleotide.

The present invention also provides a diagnostic kit for the detection of type II diabetes mellitus including the polynucleotide.

### Technical Solution

The present invention provides a method of diagnosing type II diabetes mellitus in an individual, which includes determining a nucleotide of a polymorphic site of at least one polynucleotide selected from polynucleotides identified by NCBI GenBank accession numbers in Table 1 below.

**Table 1**

| NCBI GenBank Accession No. | Polymorphic site |
|---|---|
| rs502612 | position 101 of SEQ ID NO: 1 |
| rs1394720 | position 101 of SEQ ID NO: 2 |
| rs488115 | position 101 of SEQ ID NO: 3 |
| rs2051672 | position 101 of SEQ ID NO: 4 |
| rs1038308 | position 101 of SEQ ID NO: 5 |
| rs1943317 | position 101 of SEQ ID NO: 6 |
| rs929476 | position 101 of SEQ ID NO: 7 |
| rs1984388 | position 101 of SEQ ID NO: 8 |
| rs752139 | position 101 of SEQ ID NO: 9 |
| rs2058501 | position 101 of SEQ ID NO: 10 |
| rs1059033 | position 101 of SEQ ID NO: 11 |
| rs492220 | position 101 of SEQ ID NO: 12 |
| rs1461986 | position 101 of SEQ ID NO: 13 |
| rs607209 | position 101 of SEQ ID NO: 14 |
| rs197367 | position 101 of SEQ ID NO: 15 |
| rs1340266 | position 101 of SEQ ID NO: 16 |
| rs1316909 | position 101 of SEQ ID NO: 17 |
| rs1377188 | position 101 of SEQ ID NO: 18 |

The polynucleotides of SEQ ID NOS: 1-18 are 201-bp nucleic acid fragments containing nucleotides of polymorphic sites (position 101) of rs502612, rs1394720, rs488115, rs2051672, rs1038308, rs1943317, rs929476, rs1984388, rs752139, rs2058501, rs1059033, rs492220, rs1461986, rs607209, rs197367, rs1340266, rs1316909, and rs1377188, respectively. The nucleotide sequences of SEQ ID NOS: 1-18 and characteristics of single-nucleotide polymorphisms (SNPs) present in the nucleotide sequences are summarized in Tables 2 and 3 below.

The polynucleotides of SEQ ID NOS: 1-18 are polymorphic sequences. A polymorphic sequence refers to a nucleotide sequence containing a polymorphic site at which SNP occurs. A polymorphic site refers to a position of a polymorphic sequence at which SNP occurs. The polynucleotides of SEQ ID NOS: 1-18 may be DNAs or RNAs.

An embodiment of the present invention provides a method of diagnosing type II diabetes mellitus using a multilocus marker including polymorphic sites (position 101) of two or more polymorphic sequences selected from polymorphic sequences of SEQ ID NOS: 1-18 associated with type II diabetes mellitus. The multilocus marker was identified by DNA sequence analysis of blood samples obtained from type II diabetes mellitus patients and normal persons. Characteristics of the polymorphic sequences of SEQ ID NOS: 1-18 are summarized in Tables 2-3.

**Table 2**

| ASSAY_ID | SNP | | SNP sequence (SEQ ID NO.) | Allele frequency | | | Number of Genotype | | | | | |
|---|---|---|---|---|---|---|---|---|---|---|---|---|
| | A1 | A2 | | cas_A2 | con_A2 | Delta | cas_A1A1 | cas_A1A2 | cas_A2A2 | con_A1A1 | con_A1A2 | con_A2A2 |
| DMX_001 | C | T | 1 | 0.592 | 0.492 | 0.1 | 54 | 136 | 109 | 77 | 151 | 72 |
| DMX_009 | T | G | 2 | 0.664 | 0.737 | 0.073 | 31 | 138 | 129 | 19 | 119 | 161 |
| DMX_011 | A | G | 3 | 0.866 | 0.931 | 0.065 | 7 | 66 | 225 | 1 | 39 | 258 |
| DMX_029 | C | A | 4 | 0.057 | 0.104 | 0.047 | 268 | 28 | 3 | 241 | 52 | 5 |
| DMX_030 | C | T | 5 | 0.077 | 0.129 | 0.052 | 251 | 41 | 2 | 221 | 70 | 3 |
| DMX_032 | T | A | 6 | 0.718 | 0.593 | 0.125 | 26 | 117 | 157 | 51 | 142 | 107 |
| DMX_033 | T | C | 7 | 0.816 | 0.9 | 0.084 | 10 | 89 | 198 | 4 | 51 | 239 |
| DMX_044 | A | T | 8 | 0.846 | 0.787 | 0.059 | 7 | 78 | 213 | 15 | 93 | 181 |
| DM _056 | A | G | 9 | 0.362 | 0.273 | 0.089 | 123 | 137 | 40 | 160 | 116 | 24 |
| DMX_062 | C | T | 10 | 0.421 | 0.508 | 0.087 | 106 | 133 | 59 | 72 | 146 | 77 |
| DMX_069 | T | C | 11 | 0.44 | 0.498 | 0.058 | 96 | 143 | 60 | 66 | 164 | 65 |
| DMX_104 | T | C | 12 | 0.274 | 0.204 | 0.07 | 158 | 115 | 24 | 184 | 95 | 12 |
| DMX_116 | T | C | 13 | 0.6 | 0.668 | 0.068 | 41 | 157 | 101 | 29 | 139 | 129 |
| DMX_152 | A | C | 14 | 0.562 | 0.64 | 0.078 | 62 | 136 | 99 | 41 | 129 | 123 |
| DMX_154 | A | G | 15 | 0.269 | 0.199 | 0.07 | 153 | 131 | 15 | 187 | 100 | 9 |
| DMX_058 | A | G | 16 | 0.315 | 0.382 | 0.067 | 138 | 131 | 28 | 111 | 144 | 41 |
| DMX_101 | A | T | 17 | 0.38 | 0.316 | 0.064 | 118 | 136 | 46 | 138 | 133 | 28 |
| DMX _131 | A | T | 18 | 0.441 | 0.376 | 0.065 | 97 | 139 | 62 | 118 | 136 | 44 |

**Table 2 (continued)**

| df=2 | | Odds ratio (multiple model) | | HWE status | | Sample call rate | |
|---|---|---|---|---|---|---|---|
| Chi_square value | Chi_exact_p-Value | OR | CI | con_HW | cas_HW | cas_call_rate | con_call_rate |
| 12.384 | 2.05E-03 | 0.67 | (0.53, 0.838) | .027, HWE | 1.195, HWE | 1 | 1 |
| 7.814 | 2.01E-02 | 1.42 | (1.106,1. 82) | .195, HWE | .424, HWE | 0.99 | 1 |
| 13.698 | 1.06E-03 | 2.10 | (1.414, 3.115) | .026, HWE | .948, HWE | 0.99 | 0.99 |
| 9.131 | 1.04E-02 | 1.93 | (1.247, 2.975) | 1.514, HWE | 13.034, HWD | 1 | 0.99 |
| 9.683 | 7.89E-03 | 1.79 | (1.215, 2.64) | .51, HWE | 1.004, HWE | 0.98 | 0.98 |
| 20 | 4.54E-05 | 0.57 | (0.449, 0.728) | .148, HWE | .582, HWE | 1 | 1 |
| 16.718 | 2.34E-04 | 2.02 | (1.434, 2.831) | 2.023, HWE | .005, HWE | 0.99 | 0.98 |
| 6.687 | 3.53E-02 | 0.68 | (0.501, 0.91) | .452, HWE | .013, HWE | 0.99 | 0.96 |
| 10.581 | 5.04E-03 | 0.66 | (0.52, 0.848) | .283, HWE | .041, HWE | 1 | 1 |
| 9.468 | 8.79E-03 | 1.42 | (1.131, 1.788) | .034, HWE | 2.43, HWE | 0.99 | 0.98 |
| 7.165 | 2.78E-02 | 1.27 | (1.007, 1.59) | 3.708, HWE | .364, HWE | 1 | 0.98 |
| 7.821 | 2.00E-02 | 0.68 | (0.519, 0.891) | .011, HWE | .284, HWE | 0.99 | 0.97 |
| 6.554 | 3.77E-02 | 1.34 | (1.059, 1.7) | .838, HWE | 2.473, HWE | 1 | 0.99 |
| 7.034 | 2.97E-02 | 1.38 | (1.095, 1.748) | .774, HWE | 1.715, HWE | 0.99 | 0.98 |
| 9.045 | 1.09E-02 | 0.68 | (0.515, 0.886) | .768, HWE | 3.616, HWE | 1 | 0.99 |
| 5.99 | 5.00E-02 | 1.34 | (1.057, 1.708) | 0.308, HWE | 0.112, HWE | 0.99 | 0.99 |
| 5.973 | 5.05E-02 | 0.75 | (0.594, 0.957) | 0.166, HWB | 0.465, HWE | 1 | 1 |
| 5.14 | 7.65E-02 | 0.76 | (0.605, 0.961) | 0.194, HWE | 0.946, HWE | 0.99 | 0.99 |

**Table 3**

| ASSAY_ID | rs | SNP | | SNP sequence (SEQ ID NO) | Chromosome # | Chromosome position | Band | Gene |
|---|---|---|---|---|---|---|---|---|
| | | A1 | A2 | | | | | |
| DMX_001 | rs502612 | C | T | 1 | 1 | 167373461 | 1q24.2 | PRRX1 |
| DMX_009 | rs1394720 | T | G | 2 | 11 | 4533242 | 11p15.4 | intergenic |
| Dex_011 | rs488115 | A | G | 3 | 11 | 74409538 | 11q13.4 | intergenic |
| Dex_029 | rs2051672 | C | A | 4 | 17 | 5847149 | 17p13.2 | intergenic |
| DMX_030 | rs1038308 | C | T | 5 | 18 | 44538585 | 18q21.1 | KTAA0427 |
| DMX_032 | rs1943317 | T | A | 6 | 18 | 62419479 | 18q22.1 | intergenic |
| DMX_033 | rs929476 | T | C | 7 | 19 | 33499519 | 19q12 | intergenic |
| DMX_044 | rs1984388 | A | T | 8 | 22 | 30658575 | 22q12.3 | intergenic |
| DMX_056 | rs752139 | A | G | 9 | 5 | 175943870 | 5q35.2 | PC-LKC |
| DMX_062 | rs2058501 | C | T | 10 | 7 | 120274187 | 7q31.31 | FLJ21986 |
| DMX_069 | rs1059033 | T | C | 11 | 9 | 77736025 | 9q21.2 | GNAQ |
| DMX_1 | rs49222 | T | C | 12 | 1 | 9425459040 | 1p22.1 | ABCA4 |
| DMX_116 | rs1461986 | T | C | 13 | 13 | 75506683 | 13q22.2 | intergenic |
| DMX_152 | rs607209 | A | C | 14 | 4 | 16808165 | 4p15.32 | intergenic |
| DMX_154 | rs197367 | A | G | 15 | 7 | 36219096 | 7p14.2 | ANLN |
| DMX_058 | rs13402 66 | A | G | 16 | 6 | 102381236 | 6q16.3 | GRIK2: GRIK2 |
| DMX_101 | rs1316909 | A | T | 17 | 1 | 156770438 | 1q23.2 | |
| DMX_131 | rs1377188 | A | T | 18 | 18 | 29732602 | 18q12.1 | NOL4: NOL4 |

**Table 3 (continued)**

| ASSAY_ID | Description | SNP function | Amino acid change |
|---|---|---|---|
| DMX_001 | Paired related homeobox 1 | intron | No change |
| DMX_009 | - | intergenic | No change |
| DMX_011 | - | intergenic | No change |
| DMX_029 | - | intergenic | No change |
| DMX_030 | KIAA0427 | coding-synon | No change |
| DMX_032 | - | intergenic | No change |
| DMX_033 | - | intergenic | No change |
| DMX_044 | - | intergenic | No change |
| DMX_056 | protocadherin LKC | intron | No change |
| DMX_062 | hypothetical protein | intron | No change |
| DMX_069 | guanine nucleotide binding protein (G protein), q polypeptide | intron | No change |
| DMX_104 | ATP45; binding cassette, sub45; family A (ABC1), member 4 | intron | No change |
| DMX_116 | - | Intergenic | No change |
| DMX_152 | - | Intergenic | No change |
| DMX_154 | aniline, actin binding protein (scraps homolog, Drosophila glutamate receptor) | coding-nonsynon | No change |
| DMX_058 | ionotropic, kainate 2 | Intron | K → R |
| DMX_101 | - | - | No change |
| DMX_131 | nucleolar protein 4 | Intron | No change |

In Tables 2 and 3, the contents in columns are as defined below.

- Assay_ID represents a marker name.

- SNP is a polymorphic base of a SNP polymorphic site. Here, A1 and A2 represent respectively a low mass allele and a high mass allele as a result of sequence analysis according to a homogeneous MassExtension (hME) technique (Sequenom) and are optionally designated for convenience of experiments.

- SNP sequence represents a sequence containing a SNP site, i.e., a sequence containing allele A1 or A2 at position 101.

- In the allele frequency column, cas_A2, con_A2, and Delta respectively represent allele A2 frequency of a case group, allele A2 frequency of a normal group, and the absolute value of the difference between cas_A2 and con_A2. Here, cas_A2 is (genotype A2A2 frequency x 2 + genotype A1A2 frequency)/(the number of samples x 2) in the case group and con_A2 is (genotype A2A2 frequency x 2 + genotype A1A2 frequency)/(the number of samples x 2) in the normal group.

- Genotype frequency represents the frequency of each genotype. Here, cas_A1A1, cas_A1A2, and cas_A2A2 are the number of persons with genotypes A1A1, A1A2, and A2A2, respectively, in the case group, and con_A1A1, con_A1A2, and con_A2A2 are the number of persons with genotypes A1A1, A1A2, and A2A2, respectively, in the normal group.

- df=2 represents a chi-squared value with two degree of freedom. Chi-value represents a chi-squared value and p-value is determined based on the chi-value. Chi_exact_p-value represents p-value of Fisher's exact test of chi-square test. When the number of genotypes is less than 5, results of the chi-square test may be inaccurate. In this respect, determination of more accurate statistical significance (p-value) using the Fisher's exact test is required. The chi_exact_p-value is a variable used in the Fisher's exact test. In the present invention, when the p-value ≤ 0.05, it is considered that the genotype of the case group is different from that of the normal group, i.e., there is a significant difference between the case group and the normal group.

- Odds ratio represents the ratio of the probability of allele A1 in the case group to the probability of allele A1 in the normal group. In the present invention, the Mantel-Haenszel odds ratio method was used. CI represents a 95% confidence interval for the odds ratio and is represented by (lower limit of the confidence interval, upper limit of the confidence interval). When 1 falls under the confidence interval, it is considered that there is insignificant association of allele A1 with disease.

- HWE represents Hardy-Weinberg Equilibrium. Here, con_HWE and cas_HWE represent degree of deviation from the Hardy-Weinberg Equilibrium in the normal group and the case group, respectively. Based on chi_value=6.63 (p-value=0.01, df=1) in a chi-square (df=1) test, a value larger than 6.63 was regarded as Hardy-Weinberg Disequilibrium (HWD) and a value smaller than 6.63 was regarded as Hardy-Weinberg Equilibrium (HWE).

- Sample call rate represents the number of genotype-interpretable samples to the total number of samples used in experiments. Here, cas_call_rate and con_call_rate represent the ratio of the number of genotype-interpretable samples to the total number (300 persons) of samples used in the case group and the normal group, respectively.

Tables 2 and 3 present characteristics of SNP markers based on the NCBI build 123.

In an embodiment of the method of the present invention, when nucleotides of polymorphic sites of rs502612, rs1394720, rs488115, rs2051672, rs1038308, rs1943317, rs929476, rs1984388, rs752139, rs2058501, rs1059033, rs492220, rs1461986, rs607209, rs197367, rs1340266, rs1316909, and rs1377188 satisfy at least one of multilocus markers (1) through (7) below, it may be determined that the individual has a higher likelihood of being diagnosed as a type II diabetes mellitus patient or as at risk of developing type II diabetes mellitus:

(1) the genotype of a polymorphic site of rs488115 is AA or AG and the genotype of a polymorphic site of rs1984388 is TT;

(2) the genotype of a polymorphic site of rs2051672 is CC, the genotype of a polymorphic site of rs1943317 is AA, and the genotype of a polymorphic site of rs752139 is AG or GG;

(3) the genotype of a polymorphic site of rs1943317 is TA or AA, the genotype of a polymorphic site of rs929476 is TT or TC, and the genotype of a polymorphic site of rs1377188 is AT or TT;

(4) the genotype of a polymorphic site of rs502612 is TT, the genotype of a polymorphic site of rs2051672 is CC, the genotype of a polymorphic site of rs2058501 is CC or CT, and the genotype of a polymorphic site of rs1461986 is TT or TC;

(5) the genotype of a polymorphic site of rs1394720 is TT or TG, the genotype of a polymorphic site of rs1316909 is AT or TT, and the genotype of a polymorphic site of rs607209 is AG or GG;

(6) the genotype of a polymorphic site of rs2051672 is CC, the genotype of a polymorphic site of ras 1340266 is AA, and the genotype of a polymorphic site of rs492220 is TC or CC; and

(7) the genotype of a polymorphic site of rs1038308 is CC, the genotype of a polymorphic site of rs1059033 is TT, and the genotype of a polymorphic site of rs607209 is AA or AC.

As a result of the comparison of occurrence frequencies of the genotype patterns of the multilocus markers (1) through (7) in a patient group and a normal group, it was determined that the genotype patterns of the multilocus markers (1) through (7) were significantly associated with type II diabetes mellitus. Occurrence frequencies of the multilocus markers (1) through (7) are presented in Table 4 below.

**Table 4**

| Marker name | Genotype pattern | Occurrence frequency in patient group | Occurrence frequency in normal group | Odds ratio | 95% confidence interval |
|---|---|---|---|---|---|
| 1 | DMX_011=AA or AG and DMX_044=TT | 59 | 19 | 3.62 | (2.1, 6.24) |
| 2 | DMX_029=CC, DMX_032=AA, and DMX_056=AG or GG | 94 | 311 | 3.96 | (2.54, 6.18) |
| 3 | DMX_032=TA or AA, DMX_033=TT or TC, and DMX_131=AT or TT | 70 | 23 | 3.67 | (2.22, 6.06) |
| 4 | DMX_001=TT, DMX_029=CC, DMX_062=CC or CT, and DMX_116=TT or TC | 63 | 19 | 3.93 | (2.29, 6.76) |
| 5 | DMX_009=TT or TG, DMX_101=AT or TT, and DMX_154=AG or GG | 62 | 17 | 4.34 | (2.47, 7.62) |
| 6 | DMX_029=CC, DMX_058=AA, and DMX_104=TC or CC | 71 | 23 | 3.73 | (2.26, |
| 7 | DMX_030=CC, DMX_069=TT, and DMX_152=AA or AC | 63 | 19 | 3.93 | (2.29, 6.76) |

NCBI GenBank accession numbers corresponding to the marker names in Table 4 are as presented in Table 3. Table 4 shows occurrence frequencies of the genotype patterns of the multilocus markers (1) through (7) in 300 type II diabetes mellitus patients and 300 normal persons. 82% (247/300) of the patients satisfied at least one of the genotype patterns of the multilocus markers (1) through (7). In Table 4, the odds ratio represents the ratio of the probability of a multilocus genotype pattern in the patient group to the probability of the multilocus genotype pattern in the normal group. As shown in Table 4, all odds ratios were greater than 3.5. This reveals that occurrence frequencies of the genotype patterns of the multilocus markers (1) through (7) are closely positively associated with type II diabetes mellitus.

The method of diagnosing type II diabetes mellitus according to the present invention may include isolating a nucleic acid sample from an individual; and determining a nucleotide of at least one polymorphic site (position 101) of polynucleotides of SEQ ID NOS: 1-18 or complementary polynucleotides thereof.

The operation of isolating the nucleic acid sample from the individual may be carried out using a common DNA isolation method. For example, the nucleic acid sample can be obtained by amplifying a target nucleic acid by polymerase chain reaction (PCR) followed by purification. In addition to PCR, there may be used ligase chain reaction (LCR) (Wu and Wallace, Genomics 4, 560 (1989), Landegren et al., Science 241, 1077 (1988)), transcription amplification (Kwoh et al., Proc. Natl. Acad. Sci. USA 86, 1173 (1989)), self-sustained sequence replication (Guatelli et al., Proc. Natl. Acad. Sci. USA 87, 1874 (1990)), or nucleic acid sequence based amplification (NASBA). The last two methods are related to isothermal reaction based on isothermal transcription and produce 30 or 100-fold RNA single strands and DNA double strands as amplification products.

The operation of determining the nucleotide of the at least one polymorphic site may be carried out using any method known in the art. For example, the dideoxy method for direct nucleotide sequence determination or the hybridization method for indirect nucleotide sequence determination may be used. For the latter, various methods may be used. For example, a nucleic acid microarray may be used. That is, the operation of determining the nucleotide of the at least one polymorphic site may include hybridizing the nucleic acid sample onto a microarray immobilized with one or more polynucleotides for the diagnosis or treatment of type II diabetes mellitus, each of which includes at least 10 contiguous nucleotides derived from the group consisting of nucleotide sequences of SEQ ID NOS: 1-18 and includes a nucleotide of the position 101, or complementary polynucleotides thereof; and detecting the hybridization result.

A microarray and a method of manufacturing a microarray by immobilizing a probe polynucleotide on a substrate are well known in the art. Immobilization of a probe polynucleotide associated with type II diabetes mellitus of the present invention on a substrate can be easily performed using a conventional technique. Hybridization of nucleic acids on a microarray and detection of the hybridization result are also well known in the art. For example, the detection of the hybridization result can be performed by labeling a nucleic acid sample with a labeling material generating a detectable signal, such as a fluorescent material (e.g., Cy3 and Cy5), hybridizing the labeled nucleic acid sample onto a microarray, and detecting a signal generated from the labeling material.

The present invention also provides a polynucleotide including at least 10 contiguous nucleotides of at least one nucleotide sequence selected from the group consisting of polymorphic sequences of Table 5 below and including a nucleotide of a polymorphic site (position 101) of the at least one nucleotide sequence, or a complementary polynucleotide thereof.

**Table 5**

| NCBI GenBank Accession No. | Polymorphic site | Polymorphic base |
|---|---|---|
| rs502612 | position 101 of SEQ ID NO: 1 | C or T |
| rs1394720 | position 101 of SEQ ID NO:2 | T or G |
| rs488115 | position 101 of SEQ ID NO:3 | A or G |
| rs2051672 | position 101 of SEQ ID NO:4 | C or A |
| rs1038308 | position 101 of SEQ ID NO:5 | C or T |
| rs1943317 | position 101 of SEQ ID NO:6 | T or A |
| rs929476 | position 101 of SEQ ID NO:7 | T or C |
| rs1984388 | position 101 of SEQ ID NO:8 | A or T |
| rs752139 | position 101 of SEQ ID NO:9 | A or G |
| rs2058501 | position 101 of SEQ ID NO: 10 | C or T |
| rs1059033 | position 101 of SEQ ID NO:11 | T or C |
| rs492220 | position 101 of SEQ ID NO: 12 | T or C |
| rs1461986 | position 101 of SEQ ID NO: 13 | T or C |
| rs607209 | position 101 of SEQ ID NO: 14 | A or C |
| rs197367 | position 101 of SEQ ID NO: 15 | A or G |
| rs1340266 | position 101 of SEQ ID NO: 16 | A or G |
| rs1316909 | position 101 of SEQ ID NO: 17 | A or T |
| rs1377188 | position 101 of SEQ ID NO: 18 | A or T |

The polynucleotide may be at least one polynucleotide set selected from the group consisting of polynucleotide sets (1) through (7) below:

(1) rs488115 and rs1984388;

(2) rs2051672, rs1943317, and rs752139;

(3) rs1943317, rs929476, and rs1377188;

(4) rs502612, rs2051672, rs2058501, and rs1461986;

(5) rs1394720, rs1316909, and rs197367;

(6) rs2051672, rs1340266, and rs492220; and

(7) rs1038308, rs1059033, and rs607209.

The polynucleotide of the present invention can be used as a primer or a probe. The polynucleotide can be immobilized onto a solid substrate, i.e., a microarray, as well as in a solution. Since the polynucleotide of the present invention is a type II diabetes mellitus-specific nucleotide sequence, it can be used for type II diabetes mellitus-related applications such as diagnosis or treatment of type II diabetes mellitus.

The present invention also provides a microarray immobilized with the polynucleotide of the present invention. The polynucleotide and the microarray are as described above.

The present invention also provides a diagnostic kit for the detection of type II diabetes mellitus including the polynucleotide of the present invention. Preferably, the diagnostic kit includes at least one multilocus marker polynucleotide.

In the diagnostic kit of the present invention, the polynucleotide contained in the diagnostic kit is as described above. The diagnostic kit of the present invention may include the manufacturer's specification stating a method, materials, etc. to an extent that can be understood by those of ordinary skill in the art. For example, the diagnostic kit can be used in identifying a predetermined allele at a polymorphic site by hybridizing a nucleic acid sample obtained from an individual onto the polynucleotide of the present invention used as a probe and measuring the degree of hybridization using a signal generated from the resultant hybrids. Based on the identification of predetermined allele or genotype, it can be determined if an individual has a likelihood of being diagnosed as at risk of developing type II diabetes mellitus or as a type II diabetes mellitus patient.

### Advantageous Effects

According to a method of diagnosing type II diabetes mellitus of the present invention, the presence or a risk of type II diabetes mellitus can be effectively detected.

### Best Mode

Hereinafter, the present invention will be described more specifically by Examples. However, the following Examples are provided for illustrative purposes only and are not intended to limit the scope of the present invention.

**Examples**

**Example 1**

In Example 1, DNA samples were extracted from blood of a patient group consisting of 300 Korean persons that had been identified as type II diabetes mellitus patients and had been undergoing treatment and a normal group consisting of 300 persons free from symptoms of type II diabetes mellitus and being of the same age as the patient group, and occurrence frequencies of specific SNPs were evaluated. The SNPs used in this Example were selected from a known database (NCBI dbSNP:http://www.ncbi.nlm.nih.gov/SNP/) or (Sequenom:http://www.realsnp.com/). Primers hybridizing with sequences around the selected SNPs were used to assay the nucleotide sequences of SNPs in the DNA samples.

1. Preparation of DNA samples

DNA samples were extracted from blood of type II diabetes mellitus patients and normal persons. The DNA extraction was performed according to a known extraction method (Molecular cloning: A Laboratory Manual, p 392, Sambrook, Fritsch and Maniatis, 2nd edition, Cold Spring Harbor Press, 1989) and the specification of a commercial kit manufactured by Centra system. Among extracted DNA samples, only DNA samples having a purity (A₂₆₀/A₂₈₀nm) of at least 1.7 were used.

2. Amplification of target DNAs

Target DNAs, which were predetermined DNA regions containing SNPs to be analyzed, were amplified by PCR. The PCR was performed using a common method under the following conditions. First, 2.5 ng/ml of target genomic DNAs were prepared. Then, the following PCR mixture was prepared.

Water (HPLC grade) 2.24 □

10x buffer (15 mM MgCl₂, 25 mM MgCl₂)0.5 □

dNTP Mix (GIBCO) (25 mM for each) 0.04 □

Taq pol (HotStar) (5U/□) 0.02 □

Forward/reverse primer Mix (1 µ M for each) 0.02 □

DNA 1.00 □

Total volume 5.00 □

Here, the forward and reverse primers were designed based on upstream and downstream sequences of SNPs of a known database. These primers are listed in Table 6 below.

The thermal cycles of PCR were as follows: incubation at 95 °C for 15 minutes; 45 cycles at 95 °C for 30 seconds, at 56 °C for 30 seconds, and at 72 °C for 1 minute; and incubation at 72 °C for 3 minutes and storage at 4 °C. As a result, amplified DNA fragments which were 200 or less nucleotides in length were obtained.

3. Analysis of SNPs in amplified target DNA fragments

Analysis of SNPs in the amplified target DNA fragments was performed using a homogeneous MassEXTEND (hME) technique available from Sequenom. The principle of the MassEXTEND technique is as follows. First, primers (also called 'extension primers') ending immediately before SNPs within the target DNA fragments were designed. Then, the primers were hybridized with the target DNA fragments and DNA polymerization was performed. At this time, a polymerization solution contained a reagent (e.g., ddTTP) terminating the polymerization immediately after the incorporation of a nucleotide complementary to a first allelic nucleotide (e.g., A allele). In this regard, when the first allele (e.g., A allele) exists in the target DNA fragments, products in which only a nucleotide (e.g., T nucleotide) complementary to the first allele is extended from the primers will be obtained. On the other hand, when a second allele (e.g., G allele) exists in the target DNA fragments, a nucleotide (e.g., C nucleotide) complementary to the second allele is added to the 3'-ends of the primers and then the primers are extended until a nucleotide complementary to the closest first allele nucleotide (e.g., A nucleotide) is added. The lengths of products extended from the primers were determined by mass spectrometry. Therefore, alleles present in the target DNA fragments could be identified. Illustrative experimental conditions were as follows.

First, unreacted dNTPs were removed from the PCR products. For this, 1.53 0 of deionized water, 0.17 □ of hME buffer, and 0.30 0 of shrimp alkaline phosphatase (SAP) were added and mixed in 1.5 ml tubes to prepare SAP enzyme solutions. The tubes were centrifuged at 5,000 rpm for 10 seconds. Thereafter, the PCR products were added to the SAP solution tubes, sealed, incubated at 37°C for 20 minutes and then at 85 °C for 5 minutes, and stored at 4 °C.

Next, homogeneous extension was performed using the amplified target DNA fragments as templates. The compositions of the reaction solutions for the extension were as follows.

Water (nanoscale deionized water) 1.728 □

hME extension mix (10x buffer containing 2.25 mM d/ddNTPs) 0.200 □

Extension primers (1 µ M for each) 0.054 □

Thermosequenase (32U/ □) 0.018 □

Total volume 2.00 □

The reaction solutions were thoroughly stirred and subjected to spin-down centrifugation. Tubes or plates containing the resultant solutions were compactly sealed and incubated at 94 °C for 2 minutes, followed by 40 thermal cycles at 94 °C for 5 seconds, at 52 °C for 5 seconds, and at 72 °C for 5 seconds, and storage at 4 °C . The homogeneous extension products thus obtained were washed with a resin (SpectroCLEAN^{™}). Extension primers used in the extension are listed in Table 6 below.

**Table 6: Primers for amplification and extension primers for homogeneous extension for target DNAs**

| Marker | Amplification primer (SEQ ID NO) | | Extension primer (SEQ ID NO) |
|---|---|---|---|
| | Forward primer | Reverse primer | |
| DMX_001 | 19 | 20 | 21 |
| DMX_009 | 22 | 23 | 24 |
| DMX_011 | 25 | 26 | 27 |
| DMX_029 | 28 | 29 | 30 |
| DMX_030 | 31 | 32 | 33 |
| DMX_032 | 34 | 35 | 36 |
| DMX_033 | 37 | 38 | 39 |
| DMX_044 | 40 | 41 | 42 |
| DMX_056 | 43 | 44 | 45 |
| DMX_062 | 46 | 47 | 48 |
| DMX_069 | 49 | 50 | 51 |
| DMX_104 | 52 | 53 | 54 |
| DMX_116 | 55 | 56 | 57 |
| DMX_152 | 58 | 59 | 60 |
| DMX_154 | 61 | 62 | 63 |
| DMX_058 | 64 | 65 | 66 |
| DMX_101 | 67 | 68 | 69 |
| DMX_131 | 70 | 71 | 72 |

Nucleotides of polymorphic sites in the extension products were assayed using mass spectrometry, MALDI-TOF (Matrix Assisted Laser Desorption and Ionization-Time of Flight). The MALDI-TOF is operated according to the following principle. When an analyte is exposed to a laser beam, it flies toward a detector positioned at the opposite side in a vacuum state, together with an ionized matrix. At this time, the time taken for the analyte to reach the detector is calculated. A material with a smaller mass reaches the detector more rapidly. The nucleotides of SNPs in the target DNA fragments are determined based on a difference in mass between the DNA fragments and known SNP sequences.

The results for the determination of polymorphic sequences of the target DNAs using the MALDI-TOF are shown in Tables 2 and 3. Each allele may exist in the form of homozygote or heterozygote in an individual. According to Mendel's Law of inheritance and Hardy-Weinberg Law, a genetic makeup of alleles constituting a population is maintained at a constant frequency. When the genetic makeup is statistically significant, it can be considered to be biologically meaningful. The SNPs according to the present invention occur in type II diabetes mellitus patients at a statistically significant level, as shown in Tables 2 and 3, and thus, can be efficiently used in diagnosis of type II diabetes mellitus.

As shown in Table 4, genotype patterns based on the combination of the nucleotides of the polymorphic sites of Tables 2 and 3, i.e., multilocus genotype patterns are highly associated with type II diabetes mellitus.

### Industrial Applicability

According to a method of diagnosing type II diabetes mellitus of the present invention, the presence or a risk of type II diabetes mellitus can be effectively detected.

The present application in particular refers to the following items:
1) A method of diagnosing type II diabetes mellitus in an individual, which comprises determining a nucleotide of a polymorphic site of at least one polynucleotide selected from polynucleotides identified by NCBI GenBank accession numbers in a table below.

| NCBI GenBank Accession No. | Polymorphic site |
|---|---|
| rs502612 | position 101 of SEQ ID NO: 1 |
| rs1394720 | position 101 of SEQ ID NO: 2 |
| rs488115 | position 101 of SEQ ID NO: 3 |
| rs2051672 | position 101 of SEQ ID NO: 4 |
| rs1038308 | position 101 of SEQ ID NO: 5 |
| rs1943317 | position 101 of SEQ ID NO: 6 |
| rs929476 | position 101 of SEQ ID NO: 7 |
| rs1984388 | position 101 of SEQ ID NO: 8 |
| rs752139 | position 101 of SEQ ID NO: 9 |
| rs2058501 | position 101 of SEQ ID NO: 10 |
| rs1059033 | position 101 of SEQ ID NO: 11 |
| rs492220 | position 101 of SEQ ID NO: 12 |
| rs1461986 | position 101 of SEQ ID NO: 13 |
| rs607209 | position 101 of SEQ ID NO: 14 |
| rs197367 | position 101 of SEQ ID NO: 15 |
| rs1340266 | position 101 of SEQ ID NO: 16 |
| rs1316909 | position 101 of SEQ ID NO: 17 |
| rs1377188 | position 101 of SEQ ID NO: 18 |

2) The method of item 1, wherein when the nucleotides of the polymorphic sites of SEQ ID NOS: 1-18 satisfy at least one of multilocus markers (1) through (7) below, it is determined that the individual has a higher likelihood of being diagnosed as a type II diabetes mellitus patient or as at risk of developing type II diabetes mellitus:
(1) the genotype of a polymorphic site of rs488115 is AA or AG and the genotype of a polymorphic site of rs1984388 is TT;
(2) the genotype of a polymorphic site of rs2051672 is CC, the genotype of a polymorphic site of rs1943317 is AA, and the genotype of a polymorphic site of rs752139 is AG or GG;
(3) the genotype of a polymorphic site of rs1943317 is TA or AA, the genotype of a polymorphic site of rs929476 is TT or TC, and the genotype of a polymorphic site of rs 1377188 is AT or TT;
(4) the genotype of a polymorphic site of rs502612 is TT, the genotype of a polymorphic site of rs2051672 is CC, the genotype of a polymorphic site of rs2058501 is CC or CT, and the genotype of a polymorphic site of rs1461986 is TT or TC;
(5) the genotype of a polymorphic site of rs1394720 is TT or TG, the genotype of a polymorphic site of rs 1316909 is AT or TT, and the genotype of a polymorphic site of rs197367 is AG or GG;
(6) the genotype of a polymorphic site of rs2051672 is CC, the genotype of a polymorphic site of rs1340266 is AA, and the genotype of a polymorphic site of rs492220 is TC or CC; and
(7) the genotype of a polymorphic site of rs1038308 is CC, the genotype of a polymorphic site of rs1059033 is TT, and the genotype of a polymorphic site of rs607209 is AA or AC.
3) The method of item 1, wherein the operation of determining the nucleotide of the polymorphic site is carried out by direct nucleotide sequence analysis or hybridization.
4) The method of item 3, wherein the operation of determining the nucleotide of the polymorphic site comprises:
hybridizing a nucleic acid sample obtained from the individual onto a microarray on which a probe polynucleotide including a polymorphic site of at least one polynucleotide of SEQ ID NOS: 1-18 or a complementary probe polynucleotide thereof is immobilized; and
detecting a hybridization result.
5) A polynucleotide comprising at least 10 contiguous nucleotides of at least one nucleotide sequence selected from the group consisting of polymorphic sequences of a table below and comprising a nucleotide of a polymorphic site (position 101) of the at least one nucleotide sequence, or a complementary polynucleotide thereof.

| NCBI GenBank Accession No. | Polymorphic site | Polymorphic base |
|---|---|---|
| rs502612 | position 101 of SEQ ID NO: 1 | C or T |
| rs1394720 | position 101 of SEQ ID NO:2 | T or G |
| rs488115 | position 101 of SEQ ID NO:3 | A or G |
| rs2051672 | position 101 of SEQ ID NO:4 | C or A |
| rs1038308 | position 101 of SEQ ID NO:5 | C or T |
| rs1943317 | position 101 of SEQ ID NO:6 | T or A |
| rs929476 | position 101 of SEQ ID NO:7 | T or C |
| rs1984388 | position 101 of SEQ ID NO:8 | A or T |
| rs752139 | position 101 of SEQ ID NO:9 | A or G |
| rs2058501 | position 101 of SEQ ID NO: 10 | C or T |
| rs1059033 | position 101 of SEQ ID NO:11 | T or C |
| rs492220 | position 101 of SEQ ID NO: 12 | T or C |
| rs1461986 | position 101 of SEQ ID NO: 13 | T or C |
| rs607209 | position 101 of SEQ ID NO: 14 | A or C |
| rs197367 | position 101 of SEQ ID NO: 15 | A or G |
| rs1340266 | position 101 of SEQ ID NO: 16 | A or G |
| rs1316909 | position 101 of SEQ ID NO: 17 | A or T |
| rs1377188 | position 101 of SEQ ID NO: 18 | A or T |

6) The polynucleotide of item 5, wherein the polynucleotide is at least one polynucleotide set selected from the group consisting of polynucleotide sets (1) through (7) below:
(1) rs488115 and rs1984388;
(2) rs2051672, rs1943317, and rs752139;
(3) rs1943317, rs929476, and rs1377188;
(4) rs502612, rs2051672, rs2058501, and rs1461986;
(5) rs1394720, rs1316909, and rs197367;
(6) rs2051672, rs1340266, and rs492220; and
(7) rs1038308, rs1059033, and rs607209.
7) A microarray comprising the polynucleotide of item 5 or 6.
8) A diagnostic kit for the detection of type II diabetes mellitus comprising the polynucleotide of item 5 or 6.

## Claims

1. A method for use in diagnosing type II diabetes mellitus in an individual, which comprises determining a nucleotide of a polymorphic site of the polynucleotide identified by the NCBI GenBank accession number in the table below:
| NCBI GenBank Accession No. | Polymorphic site |
|---|---|
| rs1943317 | Position 101 of SEQ ID NO: 6 |

2. The method of claim 1, further comprising determining the nucleotide of a further polymorphic site of at least one, preferably all polynucleotides selected from the polynucleotides identified by NCBI GenBank accession numbers in the table below:
| NCBI GenBank Accession No. | Polymorphic site |
|---|---|
| rs2051672 | Position 101 of SEQ ID NO: 4 |
| rs752139 | Position 101 of SEQ ID NO: 9 |

3. The method of claim 1 or 2, wherein when the nucleotides of the polymorphic sites of SEQ ID NO: 4, 6 or 9 satisfy the multilocus marker below, it is determined that the individual has a higher likelihood of being diagnosed as a type II diabetes mellitus patient or as at risk of developing type II diabetes mellitus:
the genotype of a polymorphic site of rs2051676 is CC, the genotype of a polymorphic site of rs1943317 is AA, and/or the genotype of a polymorphic site of rs752139 is AG or GG.

4. The method of any one of claims 1 to 3, wherein the operation of determining the nucleotide of the polymorphic site is carried out by direct nucleotide sequence analysis or hybridization.

5. The method of any one of claims 1 to 3, wherein the operation of determining the nucleotide of the polymorphic site comprises:
hybridizing a nucleic acid sample obtained from the individual onto a microarray on which a probe polynucleotide including the polymorphic site of the polynucleotide of SEQ ID NO: 6 or a complementary probe polynucleotide thereof is immobilized, and
on which microarray optionally at least one, preferably all of the polynucleotides of SEQ ID NO: 4 or 9 or a complementary probe polynucleotide thereof are furthermore immobilized; and
detecting a hybridization result.

6. A polynucleotide comprising at least 10 contiguous nucleotides of the nucleotide sequence selected from the table below and comprising a nucleotide of a polymorphic site (position 101) of the nucleotide sequence, or a complementary polynucleotide thereof:
| NCBI GenBaok Accession No. | Polymorphic site | Polymorphic base |
|---|---|---|
| rs194337 | Position 101 of SEQ ID NO:6 | T or A |

7. A polynucleotide set comprising the polynucleotide of claim 6 and at least one polynucleotide comprising at least 10 contiguous nucleotides of at least one, preferably all nucleotide sequence(s) selected from the group consisting of polymorphic sequences of the table below and comprising a nucleotide of a polymorphic site (position 101) of the at least one nucleotide sequence, or a complementary polynucleotide thereof
| NCBI GenBaok Accession No. | Polymorphic site | Polymorphic base |
|---|---|---|
| rs2051672 | Position 101 of SEQ ID NO:4 | C or A |
| rs1943317 | Position 101 of SEQ ID NO:6 | T or A |
| rs752139 | Position 101 of SEQ ID NO:9 | A or G |

8. A microarray comprising the polynucleotide of claim 6 or the polynucleotide set of claim 7.

9. A diagnostic kit for the detection of type II diabetes mellitus comprising the polynucleotide of claim 6 or the polynucleotide set of claim 7.


<110>	Samsung Electronic Co., Ltd.

<120>	Method of Diagnosing Type II Diabetes Mellitus using Multilocus
	Marker, Polynucleotide including Marker Associated with Type II
	Diabetes Mellitus, and Microarray and Diagnostic Kit including
	the Polynucleotide

<130>	EP53461IFZTRpau

<140>	not yet assigned
<141>	herewith

<150>	06 715 973.1
<151>	2006-02-15

<150>	PCT/KR2006/000522
<151>	2006-02-15

<150>	KR 10-2005-0032739
<151>	2005-04-20

<150>	KR 10-2005-0012418
<151>	2005-02-15

<160>	72

<170>	KopatentIn 1.71

<210>	1
<211>	201
<212>	DNA
<213>	Homo sapiens

<220>
<221>	variation
<222>	(101)
<223>	n = C or T, polymorphic site

<400>	1
taaaaacaga tgggtgatcc cagtcctcta aatataatcg gggatgccaa atcttttcaa	60

agagaattca tatatacaac ttaaaggcca aggagcccaa ntcaatcaaa atttgagcca	120

ggatatgcta agttcaatca gcttgaatat gggcaaagtg taagacctag ccagcacttc	180

agatatatac agagaaccac a	201

<210>	2
<211>	201
<212>	DNA
<213>	Homo sapiens

<220>
<221>	variation
<222>	(101)
<223>	n= T or G, polymorphic site

<400>	2
aaactgttca gtaatcctta atgtctagtt ctttccccaa agtacaattg cctgagtaaa	60

ttatcatagg taactttgag aaggaactat gataatcatg ntatataatg aggacttttc	120

tacaaggatt caggtacctc ttcaatgagt tctagatcta gaaactgaca caagtttggg	180

aactaggcaa gaaattgtga c	201

<210>    3
<211>    201
<212>    DNA
<213>    Homo sapiens

<220>
<221>    variation
<222>    (101)
<223>    n = A or G, polymorphic site

<400>    3
tggagggtgg cagggagctg gaggagcagt gaggacttgc ttgagcagtc ttgacaagat    60

gtggcaggcc cacagccttc actgcctcta ggccccctga ntgggtcact gtggttcctt    120

cagacacaag agagacccct tattgcccca gtcccactga cagactctgc ctcccaggcc    180

cactggccct gcccagacat g    201

<210>    4
<211>    201
<212>    DNA
<213>    Homo sapiens

<220>
<221>    variation
<222>    (101)
<223>    n = C or A, polymorphic site

<400>    4
   ggggtctggg gagcagagaa accaggcatc tgtgagagag aaaaattagg acggacagag    60
   aggagcacag aggaggggtg cagggagagt ccagggggct ncattcctcc tccagctatt    120
   tatgtcttca ggcccaggtg cttcctacct atgggttctc caggttatcc ttgtgtcctc    180
   tccttgtata aactccctct t    201

<210>    5
<211>    201
<212>    DNA
<213>    Homo sapiens

<220>
<221>    variation
<222>    (101)
<223>    n= C or T, polymorphic site

<400>    5
acaaggggga ggcaggcgca caccgcaatg ccaaagagac catgaccatc gagaacccaa    60

aactggagga cactgcaggg gacaccgggc acagcagcct ngaggccccc cgcagccctg    120

acaccctggc cccggtggct tctgagcggc tgcccccaca gcagtcaggg gggcccagag    180

gttgagacaa aacgtaaaga c    201

<210>    6
<211>    201
<212>    DNA

<213>    Homo sapiens

<220>
<221>    variation
<222>    (101)
<223>    n=T or A, polymorphic site


<400>    6
acctaggcag ttttatctgt gtgcaaaatt tagaaatgtc attcctgtgg aaatgagcaa        60

atcataaata catcacagaa aagaagtcgc tatttttttg nctttaagtt gttttatagt       120

taaattgtgt cagagagttt gccatctatt ttattcctaa aaaggcctgg tggagaatat       180

atgactttcc ttgatgtaga g                                                 201


<210>    7
<211>    201
<212>    DNA
<213>    Homo sapiens

<220>
<221>    variation
<222>    (101)
<223>    n=T or C, polymorphic site


<400>    7
tgcctgtgcc atgaagctcc gcaggggccc ttccaaccct gggaatgtgt tgccaacaag        60

atcccttctg tccctgtcag gcagaggtgg cagagcactc nttggcagta agctttgtac       120

ccgaaccatt tcttttttca cagtctttag ataaggcagt ttgagttcat ttcaatagct       180

ggtacttccc gggttctgcc a                                                 201


<210>    8
<211>    201
<212>    DNA
<213>    Homo sapiens

<220>
<221>    variation
<222>    (101)
<223>    n=A or T, polymorphic site


<400>    8
agcgtagtag caaactgctg gcccacagcc tgctatgaag taggagttca ttaccttctt        60

cgctccaggt cttgacatgg tccaaagact tgtcttttga ngcagccctg ttgtatcctc       120

ttgagttgtc atgacattgt ctgctggtct tccagtggca aaatatccta gactttcaga       180

gctgaaaaaa aaaggtactt t                                                 201


<210>    9
<211>    201
<212>    DNA
<213>    Homo sapiens

<220>
<221>    variation
<222>    (101)

<223>    n= A or G, polymorphic site

<400>    9
ggaagcctcc tctgcccttg cctctcttgg aactcgaggt ccaccctgac aaagccacac    60

tgggtcccag ccgcagtgtc tctcctggcc cagggagcac ntacctggat cctcctcctt    120

caccgtaaag ttgtagctgg actgattgaa gatgggcagg ttatcattga tgtcctgcag    180

tcagagacag gtctgagtcc c                                              201

<210>    10
<211>    201
<212>    DNA
<213>    Homo sapiens

<220>
<221>    variation
<222>    (101)
<223>    n=C or T, polymorphic site

<400>    10
tctctgttga gtaacataac attttcatta acccttaaag gctatggagc cagaagcata    60

gcaagtaaac acccatgacc agccacttga ggtgaagaga ncagatttat ttagattttg    120

tagccatgtt gtatagttca ttcaggatct caagcttccg tacatacgat ttctcttaaa    180

tttaattctg aaaattaaaa t                                              201

<210>    11
<211>    201
<212>    DNA
<213>    Homo sapiens

<220>
<221>    variation
<222>    (101)
<223>    n=T or C, polymorphic site

<400>    11
agcgtatact tgggggcagt gagtgggcct caaagaagtg gcaaaggcag ggccctcagg    60

ctggggtgtt gaggaagtcc tagatgacaa tcgtataata nattcatata aagacagagc    120

aatagcaaca tgtcctcagc cttacaaaag gaacttaaac gtgaaatttt cttccaaatt    180

atgtggtggc aaggtcagta a                                              201

<210>    12
<211>    201
<212>    DNA
<213>    Homo sapiens

<220>
<221>    variation
<222>    (101)
<223>    n=T or C, polymorphic site

<400>    12
aggcaggctc tgaactcagg cacccccaga gctggatcct gttcgctcct cttaatggtc    60

atgcgtggga tcttatttaa cctctttaag ccctggcttc ncatctgcaa attggcaatg 120

ataatggtgc aagcctcatg gagctgtgag aattaaatga agcatatgtg tgtaaaagca 180

gttggcacag tacttggcat a 201

<210> 13
<211> 201
<212> DNA
<213> Homo sapiens

<220>
<221> variation
<222> (101)
<223> n=T or C, polymorphic site

<400> 13
ttcttctttc ctttcaaagg catttactta aaccagactt ttccctcatc tctctcgatc 60

actttggaga ctcaagctaa catactaact cttgctttca nacacacttt ctgtttcttc 120

ttcctgtagt taataacatc ggatggagtg tgttgtaata aatacaattg agggccagga 180

gcaatggctc actcctgtaa t 201

<210> 14
<211> 201
<212> DNA
<213> Homo sapiens

<220>
<221> variation
<222> (101)
<223> n=A or C, polymorphic site

<400> 14
cagcattgat tgactcccgt gagcaaagaa gccattagca ctctcacact tcttccctcc 60

aaacctcccg ccacctccca gttttcgtta gctagagcat nattttactc tgtcaggact 120

gtagtatttg cattctgctt tgtagcaata attctcaaca attttttagt attaagtcta 180

tatttaaatg gattcaatgt t 201

<210> 15
<211> 201
<212> DNA
<213> Homo sapiens

<220>
<221> variation
<222> (101)
<223> n=A or G, polymorphic site

<400> 15
atggttttta actcttttct gaaaacattt tcagatgaca ttcctgaaag ctcactcttc 60

tcaccaatgc catcagagga aaaggctgct tcccctccca nacctctgct ttcaaatgcc 120

tcggcaactc cagttggcag aaggggccgt ctggccaatc ttgctgcaac tatttgctcc 180

tgggaagatg atgtaaatca c                                              201

<210>    16
<211>    201
<212>    DNA
<213>    Homo sapiens

<220>
<221>    variation
<222>    (101)
<223>    n=A or G, polymorphic site

<400>    16
tttggagatt agctaatagg tacagtctcc tttcccatca aaccttttac tggaaccaca      60

atgaaatagt acatttgatt gattcaaact tcagcttttt ntttcttact atctttccct      120

tgtaaattaa actacccaaa attttaatga ccaaaataga aattatcagc agaaagatag      180

taacaaccaa ctgaaggaat t                                              201

<210>    17
<211>    201
<212>    DNA
<213>    Homo sapiens

<220>
<221>    variation
<222>    (101)
<223>    n=A or T, polymorphic site

<400>    17
   acccactcta cagagatctg agctctgcag ggcccctccg tgataaacag aatcagtcag      60
   agggggactt aactcagtgg ctaaggactt ttctccaagt ntggaggtcc tgataaccct      120
   gacttcatta accctttatt gccctagcca gtggggtggg agggagagat tgtttcctgc      180
   ccttttttcg ttgttatatc a                                              201

<210>    18
<211>    201
<212>    DNA
<213>    Homo sapiens

<220>
<221>    variation
<222>    (101)
<223>    n=A or T, polymorphic site

<400>    18
attttttctt ctcttagtac cacttccttc agtcaaataa tttccaagtc ctgtcagtga      60

catgttgaaa atgacaattc ttgtcatctg agaagcactt nttgtcaatg cgtaacttca      120

aagctcatca tctctcattt gaattattgt gataacatac caatcctgtt tcttttttca      180

tattcacatc ttcttaagtt a                                              201

<210>    19
<211>    30

<212>	DNA
<213>	Artificial Sequence

<220>
<223>	primer

<400>	19
acgttggatg atacaactta aaggccaagg	30

<210>	20
<211>	30
<212>	DNA
<213>	Artificial Sequence

<220>
<223>	primer

<400>	20
acgttggatg gcccatattc aagctgattg	30

<210>	21
<211>	20
<212>	DNA
<213>	Artificial Sequence

<220>
<223>	primer

<400>	21
ttaaaggcca aggagcccaa	20

<210>	22
<211>	30
<212>	DNA
<213>	Artificial Sequence

<220>
<223>	primer

<400>	22
acgttggatg cataggtaac tttgagaagg	30

<210>	23
<211>	30
<212>	DNA
<213>	Artificial Sequence

<220>
<223>	primer

<400>	23
acgttggatg aggtacctga atccttgtag	30

<210>	24
<211>	23
<212>	DNA
<213>	Artificial Sequence

<220>
<223>    primer

<400>    24
gagaaggaac tatgataatc atg                                                23

<210>    25
<211>    30
<212>    DNA
<213>    Artificial Sequence

<220>
<223>    primer

<400>    25
acgttggatg agcagtcttg acaagatgtg                                         30

<210>    26
<211>    30
<212>    DNA
<213>    Artificial Sequence

<220>
<223>    primer

<400>    26
acgttggatg tgtgtctgaa ggaaccacag                                         30

<210>    27
<211>    19
<212>    DNA
<213>    Artificial Sequence

<220>
<223>    primer

<400>    27
ctgcctctag gcccctga                                                      19

<210>    28
<211>    30
<212>    DNA
<213>    Artificial Sequence

<220>
<223>    primer

<400>    28
acgttggatg ggcctgaaga cataaatagc                                         30

<210>    29
<211>    30
<212>    DNA
<213>    Artificial Sequence

<220>
<223>    primer

<400>    29
acgttggatg aaattaggac ggacagagag                                        30

<210>    30
<211>    21
<212>    DNA
<213>    Artificial Sequence

<220>
<223>    primer

<400>    30
taaatagctg gaggaggaat g                                                 21

<210>    31
<211>    30
<212>    DNA
<213>    Artificial Sequence

<220>
<223>    primer

<400>    31
acgttggatg aacccaaaac tggaggacac                                        30

<210>    32
<211>    29
<212>    DNA
<213>    Artificial Sequence

<220>
<223>    primer

<400>    32
acgttggatg agccgctcag aagccaccg                                         29

<210>    33
<211>    17
<212>    DNA
<213>    Artificial Sequence

<220>
<223>    primer

<400>    33
accgggcaca gcagcct                                                      17

<210>    34
<211>    30
<212>    DNA
<213>    Artificial Sequence

<220>
<223>    primer

<400>    34
acgttggatg catcacagaa aagaagtcgc                                        30

<210> 35
<211> 30
<212> DNA
<213> Artificial Sequence

<220>
<223> primer

<400> 35
acgttggatg tagatggcaa actctctgac 30

<210> 36
<211> 23
<212> DNA
<213> Artificial Sequence

<220>
<223> primer

<400> 36
gaaaagaagt cgctattttt ttg 23

<210> 37
<211> 30
<212> DNA
<213> Artificial Sequence

<220>
<223> primer

<400> 37
acgttggatg ggttcgggta caaagcttac 30

<210> 38
<211> 30
<212> DNA
<213> Artificial Sequence

<220>
<223> primer

<400> 38
acgttggatg atgtgttgcc aacaagatcc 30

<210> 39
<211> 21
<212> DNA
<213> Artificial Sequence

<220>
<223> primer

<400> 39
gggtacaaag cttactgcca a 21

<210> 40

<211>	30
<212>	DNA
<213>	Artificial Sequence

<220>
<223>	primer

<400>	40
acgttggatg gaccagcaga caatgtcatg	30

<210>	41
<211>	30
<212>	DNA
<213>	Artificial Sequence

<220>
<223>	primer

<400>	41
acgttggatg ggtcttgaca tggtccaaag	30

<210>	42
<211>	19
<212>	DNA
<213>	Artificial Sequence

<220>
<223>	primer

<400>	42
gaggatacaa cagggctgc	19

<210>	43
<211>	29
<212>	DNA
<213>	Artificial Sequence

<220>
<223>	primer

<400>	43
acgttggatg tgacaaagcc acactgggt	29

<210>	44
<211>	30
<212>	DNA
<213>	Artificial Sequence

<220>
<223>	primer

<400>	44
acgttggatg tacggtgaag gaggaggatc	30

<210>	45
<211>	18
<212>	DNA
<213>	Artificial Sequence

<220>
<223>    primer

<400>    45
tcctggccca gggagcac                                                    18

<210>    46
<211>    30
<212>    DNA
<213>    Artificial Sequence

<220>
<223>    primer

<400>    46
acgttggatg aagtaaacac ccatgaccag                                       30

<210>    47
<211>    30
<212>    DNA
<213>    Artificial Sequence

<220>
<223>    primer

<400>    47
acgttggatg gcttgagatc ctgaatgaac                                       30

<210>    48
<211>    20
<212>    DNA
<213>    Artificial Sequence

<220>
<223>    primer

<400>    48
agccacttga ggtgaagaga                                                  20

<210>    49
<211>    30
<212>    DNA
<213>    Artificial Sequence

<220>
<223>    primer

<400>    49
acgttggatg taaggctgag gacatgttgc                                       30

<210>    50
<211>    30
<212>    DNA
<213>    Artificial Sequence

<220>
<223>    primer

<400>    50
acgttggatg aaagaagtgg caaaggcagg                                        30

<210>    51
<211>    19
<212>    DNA
<213>    Artificial Sequence

<220>
<223>    primer

<400>    51
gctctgtctt tatatgaat                                                    19

<210>    52
<211>    30
<212>    DNA
<213>    Artificial Sequence

<220>
<223>    primer

<400>    52
acgttggatg cttgcaccat tatcattgcc                                        30

<210>    53
<211>    30
<212>    DNA
<213>    Artificial Sequence

<220>
<223>    primer

<400>    53
acgttggatg tcttaatggt catgcgtggg                                        30

<210>    54
<211>    17
<212>    DNA
<213>    Artificial Sequence

<220>
<223>    primer

<400>    54
ttgccaattt gcagatg                                                      17

<210>    55
<211>    30
<212>    DNA
<213>    Artificial Sequence

<220>
<223>    primer

<400>    55

acgttggatg actacaggaa gaagaaacag                                    30

<210>    56
<211>    30
<212>    DNA
<213>    Artificial Sequence

<220>
<223>    primer

<400>    56
acgttggatg ctctcgatca ctttggagac                                    30

<210>    57
<211>    20
<212>    DNA
<213>    Artificial Sequence

<220>
<223>    primer

<400>    57
agaagaaaca gaaagtgtgt                                               20

<210>    58
<211>    30
<212>    DNA
<213>    Artificial Sequence

<220>
<223>    primer

<400>    58
acgttggatg atactacagt cctgacagag                                    30

<210>    59
<211>    30
<212>    DNA
<213>    Artificial Sequence

<220>
<223>    primer

<400>    59
acgttggatg acttcttccc tccaaacctc                                    30

<210>    60
<211>    22
<212>    DNA
<213>    Artificial Sequence

<220>
<223>    primer

<400>    60
tacagtcctg acagagtaaa at                                            22

<210> 61
<211> 30
<212> DNA
<213> Artificial Sequence

<220>
<223> primer

<400> 61
acgttggatg gctcactctt ctcaccaatg 30

<210> 62
<211> 30
<212> DNA
<213> Artificial Sequence

<220>
<223> primer

<400> 62
acgttggatg gttgccgagg catttgaaag 30

<210> 63
<211> 17
<212> DNA
<213> Artificial Sequence

<220>
<223> primer

<400> 63
ggctgcttcc cctccca 17

<210> 64
<211> 31
<212> DNA
<213> Artificial Sequence

<220>
<223> primer

<400> 64
acgttggatg gggtagttta atttacaagg g 31

<210> 65
<211> 30
<212> DNA
<213> Artificial Sequence

<220>
<223> primer

<400> 65
acgttggatg ctggaaccac aatgaaatag 30

<210> 66
<211> 23
<212> DNA

<213>    Artificial Sequence

<220>
<223>    primer


<400>    66
tacaagggaa agatagtaag aaa                                              23


<210>    67
<211>    30
<212>    DNA
<213>    Artificial Sequence

<220>
<223>    primer


<400>    67
acgttggatg ctggctaggg caataaaggg                                       30


<210>    68
<211>    30
<212>    DNA
<213>    Artificial Sequence

<220>
<223>    primer


<400>    68
acgttggatg tcagtggcta aggacttttc                                       30


<210>    69
<211>    20
<212>    DNA
<213>    Artificial Sequence

<220>
<223>    primer


<400>    69
cagggttatc aggacctcca                                                  20


<210>    70
<211>    30
<212>    DNA
<213>    Artificial Sequence

<220>
<223>    primer


<400>    70
acgttggatg gatgagcttt gaagttacgc                                       30


<210>    71
<211>    30
<212>    DNA
<213>    Artificial Sequence

<220>

<223>    primer

<400>    71
acgttggatg gtcctgtcag tgacatgttg                                        30

<210>    72
<211>    18
<212>    DNA
<213>    Artificial Sequence

<220>
<223>    primer

<400>    72
gaagttacgc attgacaa                                                     18